# EUROPÄISCHE PATENTSCHRIFT

(11) **EP 0 846 691 B1**
(45) Veröffentlichungstag und Bekanntmachung des Hinweises auf die Patenterteilung: **30.06.2004**
(21) Anmeldenummer: 97120714.7
(22) Anmeldetag: 26.11.1997
(51) Int. Cl.: C07D 273/00, C07D 307/82

(54) **Verfahren zur Herstellung von 3-(1-Hydroxyphenyl-1-alkoximinomethyl)dioxazinen**
Process for preparation of 3-(1-hydroxyphenyl-1-alkoximinomethyl)dioxazines
Procédé pour la préparation de 3-(-1-Hydroxyphényl-1-alkoximinométhyl)dioxazines

(30) Priorität: 09.12.1996 DE 19651039; 19.02.1997 DE 19706396
(43) Veröffentlichungstag der Anmeldung: 10.06.1998
(73) Patentinhaber: Bayer CropScience AG, 40789 Monheim (DE)
(72) Erfinder: Gallenkamp, Bernd, Dr., 42113 Wuppertal (DE); Rohe, Lothar, Dr., 42113 Wuppertal (DE); Gayer, Herbert, Dr., 40789 Monheim (DE); Gerdes, Peter, Dr., 52080 Aachen (DE)

(56) Entgegenhaltungen:
- EP-A- 0 315 886
- DE-A- 4 408 005
- DE-A- 19 602 095
- K. VENKATESWARA RAO ET AL.: PROC. INDIAN ACAD. SCI., Bd. 83A, Nr. 6, 1976, Seiten 238-42, XP002059228
- R. STOERMER ET AL.: BERICHTE DER DEUTSCHEN CHEMISCHEN GESELLSCHAFT, Bd. 35, Nr. 9, 1902, Seiten 1640-6, XP002059229
- H. LOTH ET AL.: ARCHIV DER PHARMAZIE, Bd. 306, Nr. 2, 1973, Seiten 122-6, XP002039748

## Beschreibung

Die vorliegende Erfindung betrifft ein neues Verfahren zur Herstellung von 3-(1-Hydroxyphenyl-1-alkoximinomethyl)dioxazinen, die als Vorprodukte zur Herstellung von Verbindungen mit fungiziden Eigenschaften bekannt sind (WO 95-04728). Die Erfindung betrifft ferner neue 3-(1-Hydroxyphenyl-1-alkoximinomethyl)dioxazine und neue Zwischenprodukte zu deren Herstellung und mehrere Verfahren zur Herstellung der neuen Zwischenprodukte.

Es ist bereits bekannt geworden, daß sich bestimmte 3-(1-Hydroxyphenyl-1-alkoximinomethyl)dioxazine ausgehend von den entsprechenden Hydroxyphenylessigsäureestern synthetisieren lassen (vgl. WO 95-04728). So erhält man z. B. (5,6-Dihydro[1,4,2]dioxazin-3-yl)-(2-hydroxy-phenyl)-methanon-O-methyl-oxim (1), indem man Hydroxyphenylessigsäuremethylester (a) mit Dihydropyran umsetzt, den dabei entstehenden Dihydropyranylether (b) mit t-Butylnitrit in den 2-[2-(Tetrahydropyran-2-yloxy)-phenyl]-2-hydroximino-essigsäuremethylester (c) überführt, diese Verbindung mit Iodmethan zu 2-[2-(Tetrahydropyran-2-yloxy)-phenyl]-2-methoximino-essigsäuremethylester (d) alkyliert, diesen mit Hydroxylamin zu 2-[2-(Tetrahydropyran-2-yloxy)-phenyl]-2-methoximino-N-hydroxyacetamid (e) umsetzt, letzteres mit Dibromethan zu 3-{1-[2-(Tetrahydropyran-2-yloxy)-phenyl]-1-methoximino-methyl}-5,6-dihydro-1,4,2-dioxazin (f) cyclisiert, und zum Schluß die Tetrahydropyranylgruppe säurekatalysiert abspaltet. Diese Synthese kann durch das folgende Formelschema veranschaulicht werden:

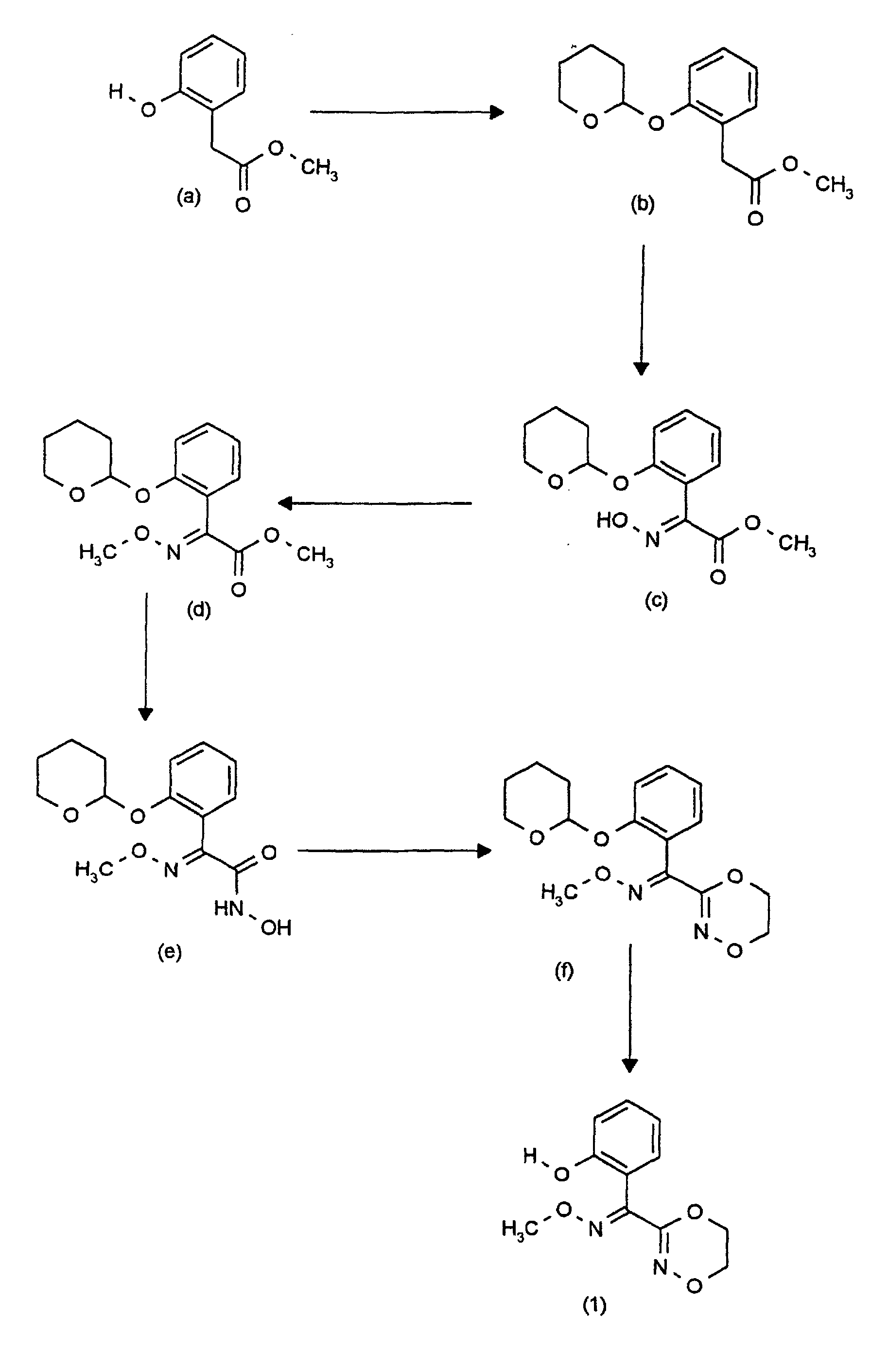

Ein entscheidender Nachteil dieses Verfahrens besteht darin, dass viele Reaktionsschritte mit teilweise geringen Ausbeuten erforderlich sind, was die Wirtschaftlichkeit dieses Verfahrens entscheidend beeinträchtigt.

Es wurde nun gefunden, dass man sowohl neue als auch bekannte 3-(1-Hydroxyphenyl-1-alkoximinomethyl)dioxazine der allgemeinen Formel (I), 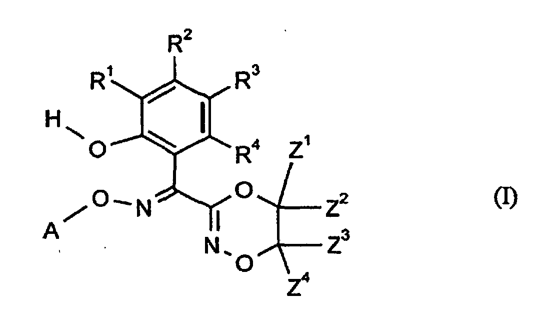 in welcher
- A: für Methyl, Ethyl, n- oder i-Propyl steht und
- R¹, R², R³ und R⁴: gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Al-Alkylsulfonyl mit jeweils 1 bis 6 Kohlenstoffatomen steht, und
- Z¹, Z², Z³ und Z⁴: gleich oder verschieden sind und unabhängig voneinander für Wasserstoff, Alkyl oder Hydroxyalkyl mit jeweils 1 bis 4 Kohlenstoffatomen oder Halogenalkyl mit 1 bis 4 Kohlenstoffatomen und 1 bis fünf gleichen oder verschiedenen Halogenatomen stehen, oder
- Z¹ und Z² oder Z¹ und Z³ oder Z³ und Z⁴: gemeinsam mit den jeweiligen Kohlenstoffatomen, an die sie gebunden sind, einen cycloaliphatischen Ring mit fünf, sechs oder sieben Kohlenstoffatomen bilden
erhält, wenn man (Verfahren a) O-Hydroxyethyl-O'-alkyl-benzofurandiondioxime der Formel (II), 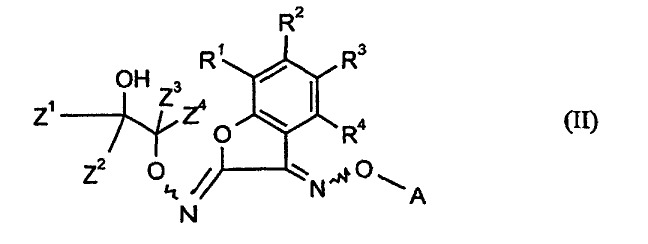 in welcher
A, R¹, R², R³, R⁴, Z¹, Z², Z³ und Z⁴ die oben angegebenen Bedeutungen haben,
gegebenenfalls in Gegenwart eines Verdünnungsmittels und gegebenenfalls in Gegenwart einer Base oder einer Säure, umlagert, und die erhaltenen Produkte, die gegebenenfalls als Gemische von Stereoisomeren vorliegen, gegebenenfalls zu den gewünschten E-Isomeren, gegebenenfalls in Gegenwart eines Verdünnungsmittels und gegebenenfalls in Gegenwart einer Säure, umisomerisiert.

In den Definitionen sind die gesättigten oder ungesättigten Kohlenwasserstoffketten, wie Alkyl, auch in Verknüpfung mit Heteroatomen, wie in Alkoxy oder Alkylthio, jeweils geradkettig oder verzweigt.

Die erfindungsgemäß herstellbaren Verbindungen und ihre Vorprodukte können gegebenenfalls als Mischungen verschiedener möglicher isomerer Formen, insbesondere von Stereoisomeren, wie z. B. E- und Z-Isomeren, gegebenenfalls aber auch von Tautomeren vorliegen. Es werden sowohl die E- als auch die Z-Isomeren, wie auch beliebige Mischungen dieser Isomeren, sowie die möglichen tautomeren Formen beansprucht.

Nach dem erfindungsgemäßen Verfahren a) werden bevorzugt Verbindungen der Formel (I) hergestellt, in welcher
- A: für Methyl oder Ethyl steht und
- R¹, R², R³ und R⁴: gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl steht,
- Z¹, Z², Z³ und Z⁴: gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
- Z¹ und Z² oder Z¹ und Z³ oder Z³ und Z⁴: gemeinsam mit den jeweiligen Kohlenstoffatomen, an die sie gebunden sind, einen cycloaliphatischen Ring mit fünf, sechs oder sieben Kohlenstoffatomen bilden.

Die oben aufgeführten allgemeinen oder in Vorzugsbereichen angegebenen Restedefinitionen gelten sowohl für die Verbindungen der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Die zur Durchführung des erfindungsgemäßen Verfahrens a) als Ausgangsstoffe benötigten O-Hydroxyethyl-O'-alkyl-benzofurandiondioxime sind durch die Formel (II) allgemein definiert. In dieser Formel (II) haben A, R¹, R², R³, R⁴, Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, R¹, R², R³, R⁴, Z¹, Z², Z³ und Z⁴ angegeben wurden. Die Ausgangsstoffe der Formel (II) sind noch nicht bekannt und als neue Stoffe ebenfalls Gegenstand der vorliegenden Anmeldung.

Die O-Hydroxyethyl-O'-alkyl-benzofurandiondioxime der Formel (II) werden erhalten, wenn man

Verfahren b) O-Alkyl-benzofurandiondioxime der Formel (III) 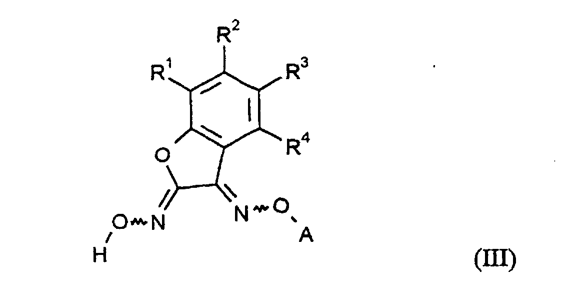 in welcher
- A, R¹, R², R³ und R⁴: die oben angegebenen Bedeutungen haben,
mit einem Ethanderivat der Formel (IV), 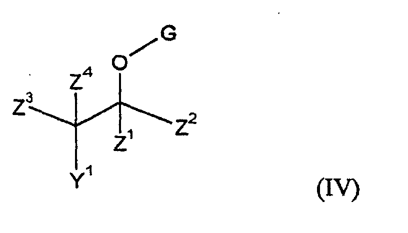 in welcher
- Y¹: für Halogen, Alkylsulfonyloxy, Arylsulfonyloxy oder Alkanoyloxy steht und
- G: für Wasserstoff steht, oder
- Y ¹ und G: durch eine Einfachbindung miteinander verbunden sind, wobei
- Y¹: für Sauerstoff steht und
- G: für 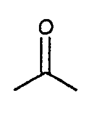 steht, oder
- Y ¹ und G: gemeinsam für eine Einfachbindung stehen und
- Z¹, Z², Z³ und Z⁴: die oben angegebenen Bedeutungen haben,
gegebenenfalls in Gegenwart eines Verdünnungsmittel, und gegebenenfalls in Gegenwart einer Base, umsetzt.

Die zur Durchführung des erfindungsgemäßen Verfahrens b) als Ausgangsstoffe benötigten O-Alkyl-benzofurandiondioxime sind durch die Formel (III) allgemein definiert. In dieser Formel (III) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Ausgangsstoffe der Formel (III) sind noch nicht bekannt und als neue Stoffe ebenfalls Gegenstand der vorliegenden Anmeldung.

Die O-Alkyl-benzofurandiondioxime der Formel (III) werden erhalten, wenn man (Verfahren c) ω-Nitro-2-hydroxyacetophenonoxime der Formel (V), 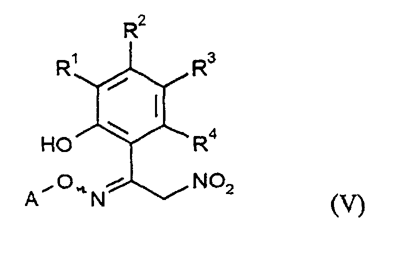 in welcher
- A, R¹, R², R³ und R⁴: die oben angegebenen Bedeutungen haben,
mit einer Base, gegebenenfalls in Gegenwart eines Verdünnungsmittels, umsetzt, oder wenn man (Verfahren d) O-Alkyl-benzofuranonoxime der Formel (VI), 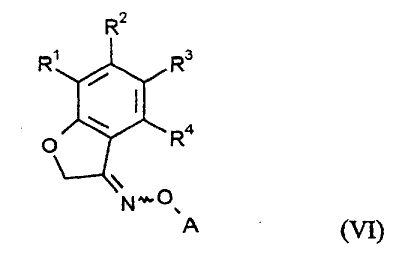 in welcher
- A, R¹, R², R³ und R⁴: die oben angegebenen Bedeutungen haben,
mit einem Alkalimetallnitrit oder einem Alkylnitrit, gegebenenfalls in Gegenwart eines Verdünnungsmittels und gegebenenfalls in Gegenwart einer Säure oder einer Base, umsetzt.

Die zur Durchführung des erfindungsgemäßen Verfahrens c) als Ausgangsstoffe benötigten ω-Nitro-2-hydroxyacetophenonoxime sind durch die Formel (V) allgemein definiert. In dieser Formel (V) haben A, R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, R¹, R², R³ und R⁴ angegeben wurden.

Die Ausgangsstoffe der Formel (V) sind noch nicht bekannt und als neue Stoffe ebenfalls Gegenstand der vorliegenden Anmeldung.

Die ω-Nitro-2-hydroxyacetophenonoxime der Formel (V) werden erhalten, wenn man (Verfahren e) ω-Nitro-2-hydroxyacetophenone der Formel (VII), 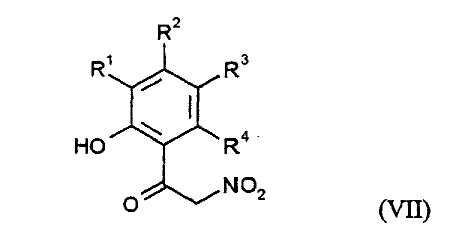 in welcher
- R¹, R², R³ und R⁴: die oben angegebenen Bedeutungen haben,
mit einem Alkoxyamin der Formel (VIII),

A-O-NH₂ (VIII)

in welcher
- A: die oben angegebene Bedeutung hat,
- oder einem Säureadditionskomplex davon -
gegebenenfalls in Gegenwart eines Verdünnungsmittel und gegebenenfalls in Gegenwart eines Säureakzeptors, umsetzt.

Die zur Durchführung des erfindungsgemäßen Verfahrens e) als Ausgangsstoffe benötigten ω-Nitro-2-hydroxyacetophenone sind durch die Formel (VII) allgemein definiert. In dieser Formel (VII) haben A, R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, R¹, R², R³ und R⁴ angegeben wurden.

Die Ausgangsstoffe der Formel (VII) sind bekannt und können nach bekannten Methoden erhalten werden (Proc.-Indian Acad.Sci.Sect.A, 83, 1976, 238,239, 242; J.Chem.Res.Miniprint, 1978, 865,877; J.Amer.Chem.Soc., 67<1945>99,101; Synthesis, 5, 1982, 397-399).

Die zur Durchführung des erfindungsgemäßen Verfahrens d) als Ausgangsstoffe benötigten O-Alkyl-benzofuranonoxime sind durch die Formel (VI) allgemein definiert. In dieser Formel (VI) haben A, R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, R¹, R², R³ und R⁴ angegeben wurden.

Die Ausgangsstoffe der Formel (VI) sind noch nicht bekannt und als neue Stoffe ebenfalls Gegenstand der vorliegenden Anmeldung.

Die O-Alkyl-benzofurandiondioxime der Formel (VI) werden erhalten, wenn man (Verfahren f) ω-Halogen-2-hydroxyacetophenonoxime der Formel (IX), 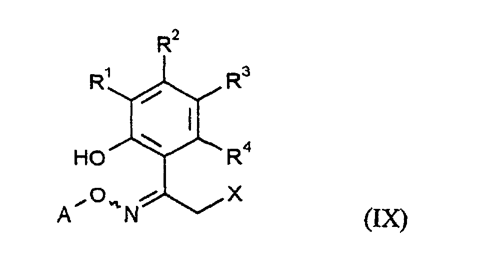 in welcher
- A, R¹, R², R³ und R⁴: die oben angegebenen Bedeutungen haben, und
- X: für Halogen steht,
mit einer Base, gegebenenfalls in Gegenwart eines Verdünnungsmittels, umsetzt, oder wenn man (Verfahren g) Benzofuranone der Formel (X) 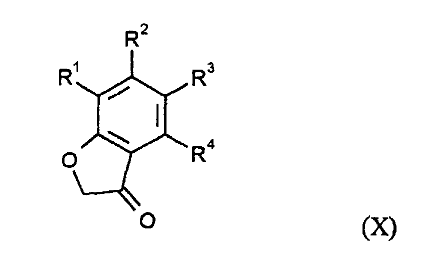 in welcher
- R¹, R², R³ und R⁴: die oben angegebenen Bedeutungen haben,
mit einem Alkoxyamin der Formel (VIII), - oder einem Säureadditionskomplex davon -
gegebenenfalls in Gegenwart eines Verdünnungsmittel und gegebenenfalls in Gegenwart eines Säureakzeptors, umsetzt, oder wenn man (Verfahren h) Benzofuranonoxime der Formel (XI) 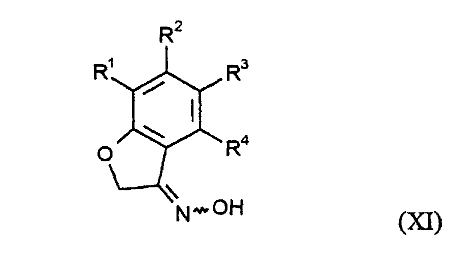 in welcher
- R¹, R², R³ und R⁴: die oben angegebenen Bedeutungen haben,
mit einem Alkylierungsmittel der Formel

A-Y (XII)

in welcher
- A: die oben angegebene Bedeutung hat und
- Y: für Halogen, Alkylsulfonyloxy, Alkoxysulfonyloxy oder Arylsulfonyloxy steht,
gegebenenfalls in Gegenwart eines Verdünnungsmittels, und gegebenenfalls in Gegenwart einer Base, umsetzt.

Die zur Durchführung des erfindungsgemäßen Verfahrens f) als Ausgangsstoffe benötigten ω-Halogen-2-hydroxyacetophenonoxime sind durch die Formel (IX) allgemein definiert. In dieser Formel (IX) haben A, R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, R¹, R², R³ und R⁴ angegeben wurden. X steht für Halogen, vorzugsweise für Chlor oder Brom.

Die Ausgangsstoffe der Formel (IX) sind noch nicht bekannt und als neue Stoffe ebenfalls Gegenstand der vorliegenden Anmeldung.

Die ω-Halogen-2-hydroxyacetophenonoxime der Formel (IX) werden erhalten, wenn man (Verfahren i) ω-Halogen-2-hydroxyacetophenone der Formel (XIII), 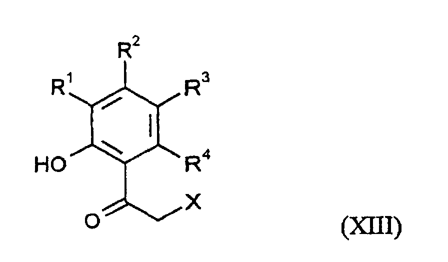 in welcher
- R¹, R², R³, R⁴ und X: die oben angegebenen Bedeutungen haben,
mit einem Alkoxyamin der Formel (VIII), - oder einem Säureadditionskomplex davon -
gegebenenfalls in Gegenwart eines Verdünnungsmittel und gegebenenfalls in Gegenwart eines Säureakzeptors, umsetzt.

Die zur Durchführung des erfindungsgemäßen Verfahrens i) als Ausgangsstoffe benötigten ω-Halogen-2-hydroxyacetophenone sind durch die Formel (XIII) allgemein definiert. In dieser Formel (XIII) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden. X steht für Halogen, vorzugsweise für Chlor oder Brom.

Die ω-Halogen-2-hydroxyacetophenone der Formel (XIII) sind bekannt und können nach bekannten Verfahren hergestellt werden (J. Org. Chem. (1990), 55(14), 4371-7 und Synthesis (1988), (7), 545-6).

Die zur Durchführung des erfindungsgemäßen Verfahrens g) als Ausgangsstoffe benötigten Benzofuranone sind durch die Formel (X) allgemein definiert. In dieser Formel (X) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Benzofuranone der Formel (X) sind bekannt und können nach bekannten Verfahren hergestellt werden (Friedlaender; Neudoerfer, Chem.Ber., 30 <1897>, 1081).

Die zur Durchführung des erfindungsgemäßen Verfahrens h) als Ausgangsstoffe benötigten Benzofuranonoxime sind durch die Formel (XI) allgemein definiert. In dieser Formel (XI) haben R¹, R², R³ und R⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für R¹, R², R³ und R⁴ angegeben wurden.

Die Benzofuranonoxime der Formel (XI) sind bekannt und können nach bekannten Verfahren hergestellt werden (vergleiche z. B. Stoermer, Bartsch, Chem.Ber., 33 <1900>, 3180).

Die weiterhin zur Durchführung des erfindungsgemäßen Verfahrens h) als Ausgangsstoffe benötigten Alkylierungsmittel sind durch die Formel (XII) allgemein definiert. In dieser Formel (XII) hat A vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für A angegeben wurde. Y steht für Halogen, vorzugsweise Chlor, Brom oder Iod, Alkylsulfonyloxy, vorzugsweise Methylsulfonyloxy, Alkoxysulfonyloxy, vorzugsweise Methoxysulfonyloxy, oder Arylsulfonyloxy, vorzugsweise 4-Tolylsulfonyloxy.

Die Alkylierungsmittel der Formel (XII) sind bekannte Synthesechemikalien.

Die weiterhin zur Durchführung der erfindungsgemäßen Verfahren e), g) und i) als Ausgangsstoffe benötigten Alkoxyamine sind durch die Formel (VIII) allgemein definiert. In dieser Formel (VIII) hat A vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für A angegeben wurde. Bevorzugte Säureadditionskomplexe der Alkoxyamine der Formel (VIII) sind deren Hydrochloride, Sulfate und Hydrogensulfate.

Die Alkoxyamine der Formel (VIII) und deren Säureadditionskomplexe sind bekannte Synthesechemikalien.

Die weiterhin zur Durchführung des erfindungsgemäßen Verfahrens b) als Ausgangsstoffe benötigten Ethanderivate sind durch die Formel (IV) allgemein definiert. In dieser Formel (IV) haben Z¹, Z², Z³ und Z⁴ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für Z¹, Z², Z³ und Z⁴ angegeben wurden. Y¹ steht für Halogen, vorzugsweise Chlor, Brom oder Iod, Alkylsulfonyloxy, vorzugsweise Methylsulfonyloxy, Arylsulfonyloxy, vorzugsweise 4-Tolylsulfonyloxy, oder Alkanoyloxy, vorzugsweise Acetyloxy. G steht für Wasserstoff oder ist durch eine Einfachbindung mit Y¹ verbunden, wobei Y¹ für Sauerstoff steht und G für Carbonyl steht, oder G steht gemeinsam mit Y¹ auch für eine Einfachbindung.

Die Ethanderivate der Formel (IV), sind bekannte Synthesechemikalien.

Wird das erfindungsgemäße Verfahren a) in Gegenwart einer Säure durchgeführt, kommen als Verdünnungsmittel alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogeniert Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Dioxan, 1,2-Dimethoxyethan, 1,2-Diethoxyethan oder Anisol; Ester wie Essigsäuremethylester oder Essigsäureethylester, oder Sulfone, wie Sulfolan sowie beliebige Mischungen der genannten Verdünnungsmittel. Besonders bevorzugte Verdünnungsmittel sind Ether, wie Diethylether, 1,2-Diethoxyethan oder Anisol; aromatische Kohlenwasserstoffe, wie beispielsweise Benzol, Toluol oder Xylol.

Wird das erfindungsgemäße Verfahren a) in Gegenwart einer Base durchgeführt, kommen als Verdünnungsmittel Wasser, sowie alle organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n-oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser. Bevorzugte Verdünnungsmittel sind Wasser, Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; sowie deren Gemische mit Wasser. Besonders bevorzugte Verdünnungsmittel sind in diesem Fall Wasser oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser.

Als Verdünnungsmittel zur Durchführung des erfindungsgemäßen Verfahrens b) kommen Wasser, sowie alle organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser. Bevorzugte Verdünnungsmittel sind Wasser, Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser. Besonders bevorzugte Verdünnungsmittel sind Wasser oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser.

Als Verdünnungsmittel zur Durchführung des erfindungsgemäßen Verfahrens c) kommen Wasser, sowie alle organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser. Bevorzugte Verdünnungsmittel sind Wasser, Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; oder Nitrile, wie Acetonitril, Propionitril, n- oder i-Butyronitril oder Benzonitril, sowie deren Gemische mit Wasser.

Als Verdünnungsmittel zur Durchführung des erfindungsgemäßen Verfahrens d) kommen Wasser und alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; organische Säuren, wie Essigsäure; Ester wie Essigsäuremethylester, Essigsäureethylester oder Essigsäurebutylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Als Verdünnungsmittel zur Durchführung der erfindungsgemäßen Verfahren e, g und i) kommen alle inerten organischen Lösungsmittel in Betracht. Hierzu gehören vorzugsweise aromatische Kohlenwasserstoffe, wie beispielsweise Benzol, Toluol oder Xylol; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; organische Säuren, wie Essigsäure; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser. Besonders bevorzugte Verdünnungsmittel sind Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser. Weiterhin besonders bevorzugt sind auch Zweiphasengemische, wie beispielsweise Wasser/Toluol.

Als Verdünnungsmittel zur Durchführung des erfindungsgemäßen Verfahrens f) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser, sowie Zweiphasengemische, wie beispielsweise Wasser/Toluol. Besonders bevorzugte Verdünnungsmittel sind aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; sowie Zweiphasengemische, wie beispielsweise Wasser/Toluol.

Als Verdünnungsmittel zur Durchführung der Umisomerisierung der erfindungsgemäß herstellbaren Verbindungen der Formel (I) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Dioxan, Tetrahydrofuran, 1,2- Dimethoxyethan, 1,2-Diethoxyethan oder Anisol; Ester wie Essigsäuremethylester oder Essigsäureethylester; Sulfoxide, wie Dimethylsulfoxid; oder Sulfone, wie Sulfolan.

Die Umisomerisierung der erfindungsgemäß herstellbaren Verbindungen der Formel (I) wird gegebenenfalls in Gegenwart einer Säure durchgeführt. Als solche kommen alle anorganischen und organischen Protonen- wie auch Lewissäuren infrage. Hierzu gehören beispielsweise Chlorwasserstoff, Bromwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid.

Das erfindungsgemäße Verfahren a) wird gegebenenfalls in Gegenwart einer Säure oder einer Base durchgeführt. Als Säuren kommen alle anorganischen und organischen Protonen- wie auch Lewissäuren, sowie auch alle polymeren Säure infrage. Hierzu gehören beispielsweise Chlorwasserstoff, Bromwasserstoff, Schwefelsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, saure Ionen austauscher, saure Tonerden und saures Kieselgel. Vorzugsweise kommen Chlorwasserstoff oder Bromwasserstoff infrage. Als Basen kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Besonders bevorzugte Basen sind Natrium-methylat, Natriumethylat, Kalium-tert.-butylat, Natriumhydroxid, Kaliumhydroxid, Ammoniumhydroxid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren b) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Besonders bevorzugte Basen sind Natrium-methylat, Natrium-ethylat, Kalium-tert.-butylat, Natriumhydroxid, Kaliumhydroxid, Ammoniumhydroxid, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren c) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Besonders bevorzugte Basen sind Erdalkalimetall- oder Alkalimetallhydroxide, -alkoholate, -carbonate oder -hydrogencarbonate, wie beispielsweise Natriumhydroxid, Kaliumhydroxid, Ammoniumhydroxid, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren d) wird gegebenenfalls in Gegenwart einer Säure oder einer Base durchgeführt. Als Säuren kommen alle anorganischen und organischen Protonensäuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure oder Toluolsulfonsäure. Als Basen kommen alle üblichen anorganischen oder organischen Basen infrage. Hierzu gehören vorzugsweise Erdalkalimetall- oder Alkalimetallhydride, -hydroxide oder -alkoholate, wie beispielsweise Natriumhydrid, Natrium-methylat, Natrium-ethylat, Kalium-tert.-butylat, Natriumhydroxid oder Kaliumhydroxid.

Die erfindungsgemäßen Verfahren e), g) und i) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -alkoholate, -acetate, -carbonate oder -hydrogencarbonate, wie beispielsweise Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a) und b) in einem größeren Bereich variiert werden. Im allgemeinen arbeitet man bei Temperaturen von -20°C bis 100°C, vorzugsweise bei Temperaturen von 0°C bis 80°C.

Die Reaktionstemperaturen können bei der Durchführung des erfindungsgemäßen Verfahrens c) in einem größeren Bereich variiert werden. Im allgemeinen arbeitet man bei Temperaturen von 0°C bis 150°C, vorzugsweise bei Temperaturen von 20°C bis 100°C.

Die Reaktionstemperaturen können bei der Durchführung der erfindungsgemäßen Verfahren e), g) und i) in einem größeren Bereich variiert werden. Im allgemeinen arbeitet man bei Temperaturen von 0°C bis 150°C, vorzugsweise bei Temperaturen von 0°C bis 80°C.

Die Reaktionstemperaturen können bei der Durchführung des erfindungsgemäßen Verfahrens d) in einem größeren Bereich variiert werden. Im allgemeinen arbeitet man bei Temperaturen von -20°C bis 150°C, vorzugsweise bei Temperaturen von 0°C bis 100°C.

Die Reaktionstemperaturen können bei der Durchführung des erfindungsgemäßen Verfahrens f) in einem größeren Bereich variiert werden. Im allgemeinen arbeitet man bei Temperaturen von 0°C bis 200°C, vorzugsweise bei Temperaturen von 20°C bis 150°C.

Zur Durchführung des erfindungsgemäßen Verfahrens a) zur Herstellung der Verbindungen der Formel (I) setzt man pro Mol des O-Hydroxyethyl-O'-alkyl-benzofurandiondioxims der Formel (II) im allgemeinen 1 bis 15-Mol, vorzugsweise 2 bis 6 Mol Base ein.

Zur Durchführung des erfindungsgemäßen Verfahrens b) zur Herstellung der Verbindungen der Formel (II) setzt man pro Mol des O-Alkyl-benzofurandiondioxims der Formel (III) im allgemeinen 1 bis 15 Mol, vorzugsweise 3 bis 6 Mol Ethanderivat der Formel (IV) ein.

Zur Durchführung des erfindungsgemäßen Verfahrens c) zur Herstellung der Verbindungen der Formel (III) setzt man pro Mol des ω-Nitro-2-hydroxyacetophenonoxims der Formel (V) im allgemeinen 1 bis 15 Mol, vorzugsweise 2 bis 8 Mol Base ein.

Zur Durchführung des erfindungsgemäßen Verfahrens d) zur Herstellung der Verbindungen der Formel (III) setzt man pro Mol des O-Alkyl-benzofuranonoxims der Formel (VI) im allgemeinen 1 bis 5 Mol, vorzugsweise 1 bis 3 Mol Alkalimetallnitrit oder Alkylnitrit ein.

Zur Durchführung der erfindungsgemäßen Verfahren e), g) und i) zur Herstellung der Verbindungen der Formel (V), (VI) bzw. (IX) setzt man pro Mol des ω-Nitro-2-hydroxyacetophenons der Formel (VII), bzw. des Benzofuranons der Formel (X), bzw. des ω-Halogen-2-hydroxyacetophenons der Formel (XIII) im allgemeinen 1 bis 15 Mol, vorzugsweise 1 bis 8 Mol Alkoxyamin der Formel (VIII), - oder einem Säureadditionskomplex davon - ein.

Zur Durchführung des erfindungsgemäßen Verfahrens f) zur Herstellung der Verbindungen der Formel (VI) setzt man pro Mol des ω-Halogen-2-hydroxyacetophenonoxims der Formel (IX) im allgemeinen 1 bis 15 Mol, vorzugsweise 1 bis 5 Mol Base ein.

Die erfindungsgemäßen Verfahren a) bis i) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

In einer bevorzugten Verfahrensvariante (A) wird ein ω-Nitro-2-hydroxyacetophenon der Formel (VII) durch Reaktion mit einem Alkoxyamin der Formel (VIII) - oder einem Säureadditionskomplex davon - gegebenenfalls in einem Puffersystem, wie beispielsweise Natriumacetat/Essigsäure, wie in Verfahren e) beschrieben, zu einem ω-Nitro-2-hydroxyacetophenonoxim der Formel (V) um. Dieses reagiert durch Behandlung mit einer Base, beispielsweise mit wäßriger Natriumhydrogencarbonatlösung, zu einem O-Alkyl-benzofurandiondioxim der Formel (III). Das O-Alkyl-benzofurandiondioxim der Formel (III) setzt man in basischer Lösung, beispielsweise in wäßriger Alkalimetallhydroxidlösung, mit einem Ethanderivat der Formel (IV) zu einem O-Hydroxyethyl-O'-alkyl-benzofurandiondioxim der Formel (II) um, das vorzugsweise ohne Aufarbeitung in der basischen Lösung zu dem gewünschten 3-(1-Hydroxyphenyl-1-alkoximinomethyl)dioxazin abreagiert. Dieses wird gegebenenfalls zum gewünschten E-Isomeren, beispielsweise durch Behandlung mit einer Säure, wie Chlorwasserstoff, in einem organischen Lösungsmittel, wie Essigsäureethylester, umisomerisiert.

In einer weiteren bevorzugten Verfahrensvariante (B) wird ein ω-Halogen-2-hydroxyacetophenon der Formel (XIII) durch Reaktion mit einem Alkoxyamin der Formel (VIII) - oder einem Säureadditionskomplex davon - gegebenenfalls in einem Puffersystem, wie beispielsweise Natriumacetat/Essigsäure, wie in Verfahren i) beschrieben, zu einem ω-Halogen-2-hydroxyacetophenonoxim der Formel (IX) um. Dieses cyclisiert durch Behandlung mit einer Base, beispielsweise mit Natriumhydrogencarbonat im System Wasser/Methyl-t-butylether, zu einem O-Alkyl-benzofuranonoxim der Formel (VI). Das O-Alkyl-benzofuranonoxim der Formel (VI) liefert mit einem Alkalimetall- oder Alkylnitrit, sowohl in saurer wie auch in alkalischer Lösung, ein O-Alkyl-benzofurandiondioxim der Formel (III). Das O-Alkyl-benzofurandiondioxim der Formel (III) setzt man in basischer Lösung, beispielsweise in wäßriger Alkalimetallhydroxidlösung, mit einem Ethanderivat der Formel (IV) zu einem O-Hydroxyethyl-O'-alkyl-benzofurandiondioxim der Formel (II) um, das vorzugsweise ohne Aufarbeitung in der basischen Lösung zu dem gewünschten 3-(1-Hydroxyphenyl-1-alkoximinomethyl)dioxazin abreagiert. Dieses wird gegebenenfalls zum gewünschten E-Isomeren, beispielsweise durch Behandlung mit einer Säure, wie Chlorwasserstoff, in einem organischen Lösungsmittel, wie Essigsäureethylester, umisomerisiert.

In einer dritten bevorzugten Verfahrensvariante (C) wird ein Benzofuranon der Formel (X) durch Reaktion mit einem Alkoxyamin der Formel (VIII) - oder einem Säureadditionskomplex davon - gegebenenfalls in einem Puffersystem, wie beispielsweise Natriumacetat/Essigsäure, wie in Verfahren g) beschrieben, zu einem O-Alkyl-benzofuranonoxim der Formel (VI) umgesetzt. Das O-Alkyl-benzofuranonoxim der Formel (VI) liefert mit einem Alkalimetall- oder Alkylnitrit, sowohl in saurer wie auch in alkalischer Lösung, ein O-Alkyl-benzofurandiondioxim der Formel (III). Das O-Alkyl-benzofurandiondioxim der Formel (III) setzt man in basischer Lösung, beispielsweise in wäßriger Alkalimetallhydroxidlösung, mit einem Ethanderivat der Formel (IV) zu einem O-Hydroxyethyl-O'-alkyl-benzofurandiondioxim der Formel (II) um, das vorzugsweise ohne Aufarbeitung in der basischen Lösung zu dem gewünschten 3-(1-Hydroxyphenyl-1-alkoximinomethyl)dioxazin abreagiert. Dieses wird gegebenenfalls zum gewünschten E-Isomeren, beispielsweise durch Behandlung mit einer Säure, wie Chlorwasserstoff in einem organischen Lösungsmittel, wie Essigsäureethylester, umisomerisiert.

Es ist als äußerst überraschend zu bezeichnen, daß die erfindungsgemäßen Verfahren, insbesondere in ihrer Kombination, in hoher Ausbeute und hoher Reinheit ablaufen. Besonders überraschend ist, daß Verbindungen der allgemeinen Formel (V) durch Behandlung mit Basen schon bei Raumtemperatur unverzüglich auch in Gegenwart von Wasser unter Wasserabspaltung in hohen Ausbeuten zu Verbindungen der Formel (III) cyclisieren und einfach durch Abfiltrieren in hoher Reinheit isoliert werden können. Aus der bisher bekannten Literatur wäre unter diesen Bedingungen lediglich eine Deprotonierung der Verbindungen der Formel (V) zu erwarten gewesen. Es ist weiterhin als besonders überraschend anzusehen, daß Verbindungen der Formel (III) sich ohne weiteres unter alkalischen Bedingungen mit Epoxiden zu Verbindungen der Formel (II) umsetzen lassen, die ihrerseits gegebenenfalls sogar ohne Aufarbeitung gleich zu den gewünschten 3-(1-Hydroxyphenyl-1-alkoximinomethyl)dioxazinen cyclisieren. In Chem. Ber. 1902, 1640 ist beispielsweise beschrieben, daß Benzofurandionmonooxime sowohl durch Behandlung mit Säuren, wie auch durch Behandlung mit Basen zu Salicylsäurederivaten oder Hydroxyphenylglyoxylsäurederivaten gespalten werden. Somit wäre eher damit zu rechnen gewesen, daß sich auch die Verbindungen der Formel (III) unter den angegebenen Reaktionsbedingungen zersetzen. Die glatt verlaufende Nitrosierung der Verbindungen der Formel (VI) (Verfahren d) ist ebenso als überraschend anzusehen, weil eine Nitrosierung von Methyl- oder Methylengruppen, die α-ständig zu Oximen stehen, im Gegensatz zu Methyl- oder Methylengruppen, die α-ständig zu Ketogruppen stehen, bisher nicht beschrieben wurde.

Die erfindungsgemäßen Verfahren weisen eine Reihe von Vorteilen auf So ermöglichen sie die Herstellung einer Vielzahl von 3-(1-Hydroxyphenyl-1-alkoximinomethyl)dioxazinen in hoher Ausbeute und hoher Reinheit. Günstig ist es auch, daß die als Ausgangsstoffe benötigten Ethanderivate der Formel (IV), die ω-Nitro-2-hydroxyacetophenone der Formel (VII), die Benzofuranone der Formel (X), bzw. die ω-Halogen-2-hydroxyacetophenone der Formel (XIII) in einfacher Weise auch in größeren Mengen kostengünstig zugänglich sind.

### Herstellungs- und Verfahrensbeispiele:

### Beispiel 1:

### Verfahrensvariante (A)

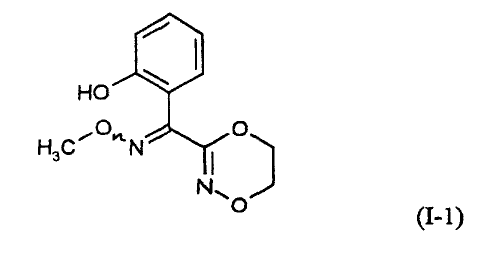

### 1 Stufe

### Verbindung (V-1)

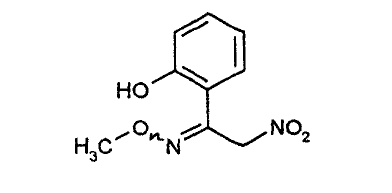

### Verfahren e)

Zu einer Lösung von 80,0 g (0,44 Mol) o-Hydroxy-ω-nitroacetophenon in 500 ml Methanol gibt man bei 20°C 73,5 g (0,88 Mol) Methoxyamin-Hydrochlorid und rührt 8 bis 12 Stunden bei 45 bis 50°C. Die Lösung wird auf 20°C abgekühlt, auf 1 l Eiswasser gegeben und eine Stunde gerührt. Das auskristallisierte Produkt wird abfiltriert, mit 500 ml Wasser portionsweise nachgewaschen und bei 40°C im Vakuumtrockenschrank getrocknet. Man erhält 72 g (74,6 % der Theorie) 1-(2-Hydroxyphenyl)-2-nitro-ethanon-O-methyl-oxim als Stereoisomerengemisch.
HPLC: logP = 1,87 (12,3 %), 2,27 (83,5 %)

### 2. Stufe

### Verbindung (III-1)

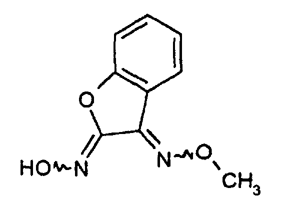

### Verfahren c)

Zu einer Lösung von 121 g (0,342 Mol) Natriumhydrogencarbonat in 700 ml Wasser gibt man 71,8 g 1-(2-Hydroxy-phenyl)-2-nitro-ethanon-O-methyl-oxim. Die Mischung wird innerhalb von 30 Minuten auf 90 bis 95°C aufgeheizt, wobei das ungelöste Ausgangsmaterial schmilzt, abreagiert und das Produkt kristallin ausfällt. Die Mischung wird auf 20°C abgekühlt und das Produkt abfiltriert, mit 500 ml Wasser portionsweise nachgewaschen und an der Luft getrocknet. Man erhält 57,4 g (91,3 % der Theorie) Benzofuran-2,3-dion-3-(O-methyl-oxim)-2-oxim als Gemisch von zwei Stereoisomeren.
HPLC: logP = 1,56 (28,4 %), 1,72 (71,6 %)

### 3. Stufe

### Verbindung (II-1 )

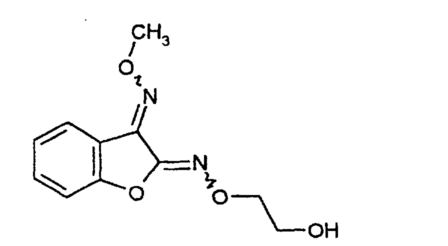

### Verfahren b)

In eine Lösung von 192,2 g (1,0 Mol) Benzofuran-2,3-dion-3-(O-methyl-oxim)-2-oxim in 2 l Wasser leitet man innerhalb von 85 Minuten bei 20°C 264,3 g (6,0 Mol) Ethylenoxid ein. Die Lösung wird auf 5°C gekühlt und 70 g (1,06 Mol) Kaliumhydroxidplätzchen zugegeben, wobei die Temperatur auf 10°C ansteigt. Es wird noch 165 Minuten ohne weitere Kühlung gerührt, der entstandene Niederschlag abgesaugt, portionsweise mit 500 ml Eiswasser gewaschen und im Vakuumtrockenschrank bei 40°C getrocknet. Man erhält 143,0 g (61 % der Theorie) Benzofuran-2,3-dion-2-[O-(2-hydroxy-ethyl)-oxim]-3-(O-methyl-oxim) als Gemisch von zwei Stereoisomeren.
HPLC: logP = 1,65 (0,5 %); 1,79 (99,5 %)

### 4. Stufe

### Verbindung (I-1)

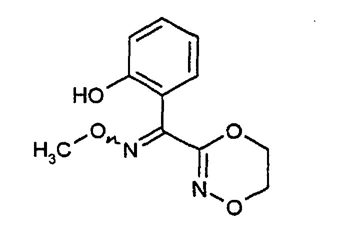

### Verfahren a)

Eine Lösung aus 25,6 g (0,1084 Mol) Benzofuran-2,3-dion-2-[O-(2-hydroxy-ethyl)-oxim]-3-(O-methyl-oxim) und 14,2 g (0,216 Mol) Kaliumhydroxid-Plätzchen in 250 ml Wasser wird 195 Minuten bei 60°C gerührt. Man kühlt die Lösung auf 10°C ab und säuert mit mit Eissessig auf einen pH-Wert von 5 - 6 an. Das auskristallisierte Produkt wird abgesaugt, portionsweise mit 200 ml Wasser gewaschen und bei 45°C im Vakuumtrockenschrank getrocknet. Man erhält 17,7g (67,7 % der Theorie) E-(5,6-Dihydro-[ 1,4,2]dioxazin-3-yl)-(2-hydroxy-phenyl)-methanon-O-methyl-oxim.
HPLC: logP = 1,22

### Beispiel 2

### Verbindung (I-1)

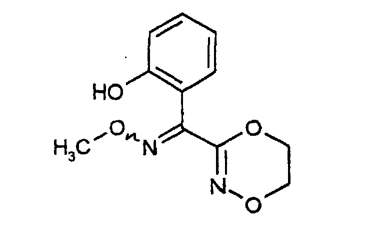

### Verfahren a) und b) als Eintopfvariante

Zu einer Suspension von 38,4 g (0,2 Mol) Benzofuran-2,3-dion-3-(O-methyl-oxim)-2-oxim in 400 ml Wasser gibt man bei 20°C 19,8 g (0,3 Mol) Kaliumhydroxidplätzchen und rührt 30 Minuten, wobei sich eine Lösung bildet. Bei 20°C leitet man innerhalb von 75 Minuten 17,6 g (0,4 Mol) Ethylenoxid ein und rührt über Nacht bei 20°C, wobei sich ein Niederschlag bildet. Die Mischung wird nun 11 Stunden bei 60°C gerührt, wobei sich der Niederschlag wieder auflöst. Man kühlt die Lösung ab und säuert mit Eissessig auf einen pH-Wert von 5 - 6 an. Das auskristallisierte Produkt wird abgesaugt, portionsweise mit 300 ml Wasser gewaschen und bei 45°C im Vakuumtrockenschrank getrocknet. Man erhält 19,6 g (39,3 % der Theorie) (5,6-Dihydro[1,4,2]dioxazin-3-yl)-(2-hydroxy-phenyl)-methanon-O-methyl-oxim.
HPLC: logP = 1,24 (E-Isomer 94,2 %); 2,05 (Z-Isomer 0,6 %)

### Beispiel 3

### Verbindung (I-1)

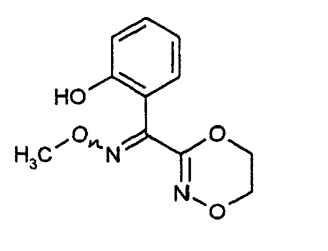

### Umisomerisierung

In eine Lösung von 806 g (4,19 Mol) (5,6-Dihydro-[1,4,2]dioxazin-3-yl)-(2-hydroxyphenyl)-methanon-O-methyl-oxim (33,3% Z-Isomer, 66,6% E-Isomer) leitet man innerhalb von 60 Minuten 112 g Chlorwasserstoff ein, wobei sich die Lösung von 20°C auf 27°C erwärmt. Die Mischung wird noch 18 Stunden bei 20°C gerührt und anschließend bei vermindertem Druck eingeengt. Der Rückstand wird bei 40°C im Vakuumtrockenschrank getrocknet. Man erhält 806 g (100 % der Theorie) (5,6-Dihydro-[1,4,2]dioxazin-3-yl)-(2-hydroxy-phenyl)-methanon-O-methyl-oxim mit einem Gehalt von 94,8% E-Isomer (HPLC).

### Beispiel 4

### Verbindung (III-1)

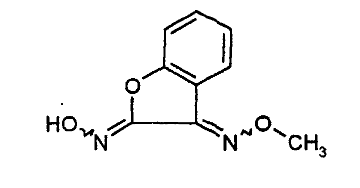

### Verfahren d)

3,92 g (0,035 Mol) Kalium-tert.-butylat werden in 40 ml tert.-Butanol gelöst. Zu dieser Lösung gibt man eine Lösung von 5,7 g (0,035 Mol) Benzofuran-3-on-O-methyloxim und 7,2 g (0,07 Mol) tert.-Butylnitrit in 10 ml tert.-Butanol. Man rührt die Mischung zwei Stunden ohne Kühlung und versetzt sie dann mit 20 ml 2N wäßriger Salzsäure. Man filtriert das auskristallisierte Produkt ab, wäscht es mehrmals mit Wasser und trocknet es im Exsiccator. Man erhält 3,19 g (47,1 % der Theorie) Benzofuran-2,3-dion-3-(O-methyl-oxim)-2-oxim als Gemisch von zwei Stereoisomeren, bestehend aus 86,33 % Isomer A und 12,98 % Isomer B (HPLC).
¹H-NMR-Spektrum (DMSO-d₆/TMS): δ = 4,10 (3H, Isomer B); 4,11 (3H; Isomer A); 7,21/7,24/7,26 (1H); 7,31/7,34 (1H); 7,51/7,53/7,56 (1H); 7,63/7,65 (1H, Isomer B); 8,02/8,05 (1H, Isomer A); 11,36 (1H, Isomer A); 11,75 (1H, Isomer B) ppm.

Gleiche Ergebnisse werden erhalten, wenn an Stelle von tert.-Butanol Essigsäurebutylester eingesetzt wird.

### Beispiel 5

### Verbindung (III-1)

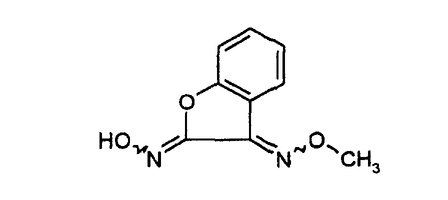

### Verfahren d)

Zu 30 ml mit trockenem Chlorwasserstoff gesättigtem Essigsäureethylester tropft man bei -10 °C 2 g (0,019 Mol) tert.-Butylnitrit und rührt 15 Minuten bei dieser Temperatur. Dann gibt man bei -10 °C 1,6 g (0,0098 Mol) Benzofuran-3-on-O-methyl-oxim, gelöst in 5 ml Essigsäureethylester zu, läßt die Temperatur auf 0 °C steigen und rührt 30 Minuten bei dieser Temperatur. Man filtriert das auskristallisierte Produkt ab und erhält 1,08 g kristallines Benzofuran-2,3-dion-3-(O-methyl-oxim)-2-oxim als Gemisch von zwei Stereoisomeren, das entsprechend der HPLC-Analyse zu 54,7 % (56 % der Theorie) aus Isomer B und zu 42,9 % aus Isomer A besteht.
¹H-NMR-Spektrum (CDCl₃/TMS): δ = 4,10 (3H, Isomer B); 4,11 (3H, Isomer A); 7,21-7,26 (1H); 7,31-7,35 (1H); 7,5-7,65 (2H, Isomer B + 1H, Isomer A); 8,02-8,05 (1H, Isomer A); 11,36 (1H, Isomer A); 11,75 (1H, Isomer B) ppm.
Analog wurde 5-Methylbenzofuran-2,3-dion-3-(O-methyl-oxim)-2-oxim (III-2) erhalten.
¹H-NMR-Spektrum (CDCl₃/TMS): δ = 2,24 (3H, Isomer B); 2,25 (3H, Isomer A) ppm.

### Beispiel 6

### Verbindung (VI-1)

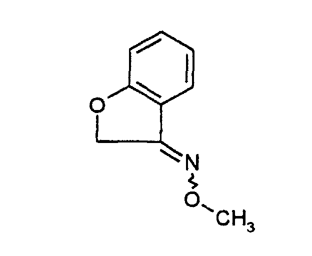

### Verfahren f)

74 g (0,303 Mol) 2-Brom-1-(2-hydroxy-phenyl)-ethanon-O-methyl-oxim werden in 350 ml tert.-Butylmethylether gelöst und mit einer Lösung von 40 g (0,377 Mol) Natriumcarbonat in 400 ml Wasser 5 Tage lang unter kräftigem Rühren unter Rückfluß gekocht. Man trennt die organische Phase ab, trocknet sie über Natriumsulfat, destilliert das Lösungsmittel im Vakuum ab und erhält 45,5 g (82,8 % der Theorie, 90 % Z-Isomer entsrechend der HPLC-Analyse) rohes Benzofuran-3-on-O-methyl-oxim.
¹H-MMR-Spektrum (DMSO-d₆/TMS): δ = 3,93 (3H); 5,16 (2H); 7,0-7,07 (2H); 7,39-7,45 (1H); 7,54-7,57 (1H) ppm.
Analog wurde 5-Methylbenzofuran-3-on-O-methyl-oxim (VI-2) erhalten.
GC/MS: M⁺ = 177; HPLC: logP = 2,88

### Beispiel 7

### Verbindung (VI-1)

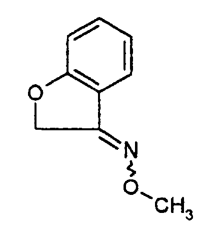

### Verfahren g)

6,7 g (0,05 Mol) Benzofuran-3-on werden mit 4,2 g (0,05 Mol) O-Methylhydroxylamin-Hydrochlorid und 4,1 g (0,05 Mol) Natriumacetat in 50 ml Methanol 3 Stunden unter Rückfluß gekocht. Man destilliert das Lösungsmittel im Vakuum ab, gießt das Reaktionsgemisch auf Wasser und extrahiert mit Essigsäureethylester. Man wäscht die organische Phase mit gesättigter, wäßriger Natriumcarbonatlösung. Man trocknet die organische Phase über Natriumsulfat und destilliert das Lösungsmittel im Vakuum ab. Man erhält 7,27 g (89,2 % der Theorie) rohes Benzofuran-3-on-O-methyl-oxim. Zur Analyse destilliert man es bei 2 Torr und 70 °C im Kugelrohr. Man erhält ein Öl, das sowohl entsprechend der NMR-Analyse als auch entsprechend der HPLC-Analyse aus zwei Stereoisomeren besteht (79 % Isomer B und 21 % Isomer A).
¹H-MMR-Spektrum (DMSO-d₆/TMS): δ = 3,93 (3H, Isomer B); 3,93 (3H, Isomer A); 5,11 (2H, Isomer A); 5,16 (2H, Isomer B); 7.0-7,07 (2H); 7,39-7,45 (1H); 7,54/7,57 (1H, Isomer B); 7,95-8,02 (1H, Isomer A) ppm.

### Beispiel 8

### Verbindung (VI-1)

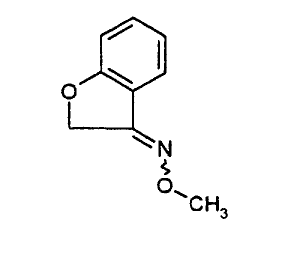

### Verfahren h)

3,7 g Benzofuran-3-on-oxim werden in 15 ml Dimethylformamid gelöst. Man gibt bei 20 °C 1 g 60 %iges Natriumhydrid zu und rührt bis zum Ende der Gasentwicklung. Dann tropft man 3,15 g Dimethylsulfat dazu und rührt 24 Stunden bei 20 °C. Man gießt das Reaktionsgemisch auf Wasser, extrahiert mit Essigsäureethylester, trocknet die organische Phase über Natriumsulfat und destilliert das Lösungsmittel im Vakuum ab. Der Rückstand wird in n-Hexan/Aceton (4:1) an Kieselgel chromatographiert. Man erhält 1,7 g (42 % der Theorie) Benzofuran-3-on-O-methyl-oxim als Öl.
¹H-NMR-Spektrum (DMSO-d₆/TMS): δ = 3,93 (3H); 5,16 (2H); 7.0-7,07 (2H); 7,39-7,45 (1H); 7,54/7,57 (1H) ppm.

### Beispiel 9

### Verbindung (IX-1)

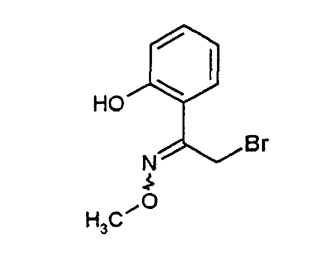

### Verfahren i)

107,5 g (0,5 Mol) ω-Brom-2-hydroxy-acetophenon werden in 500 ml Methanol mit 41,75 g (0,5 Mol) O-Methylhydroxylamin Hydrochlorid 4 Stunden unter Rückfluß gekocht. Man destilliert das Methanol im Vakuum ab, versetzt den Rückstand mit 500 ml Wasser und extrahiert mit 4 mal 100 ml Essigsäureethylester. Man trocknet die organische Phase über Natriumsulfat und destilliert das Lösungsmittel im Vakuum ab. Nach dem Verrühren mit Petrolether bei 0 °C erhält man 74 g (52 % der Theorie) kristallines 2-Brom-1-(2-hydroxy-phenyl)-ethanon-O-methyl-oxim als Gemisch von zwei Stereoisomeren (53 % Isomer B und 33 % Isomer A).
¹H-NMR-Spektrum (CDCl₃/TMS): δ = 3,98 (3H, Isomer B); 3,99 (3H, Isomer A); 4,53 (3H, Isomer A); 4,69 (3H, Isomer B); 6,85-6,93 (2H); 7,26-7,35 (2H); 10,21 (1H, Isomer B); 10,25 (1H, Isomer A) ppm.
Analog wurde 2-Chlor-1-(2-hydroxy-5-methyl-phenyl)-ethanon-O-methyl-oxim (IX-2) erhalten.
¹H-NMR-Spektrum (CDCl₃/TMS): δ = 2,32 (s, 3H); 4,08 (s, 3H) ppm.

Analog den Beispielen 1 und 2, sowie entsprechend der allgemeinen Beschreibung des erfindungsgemäßen Verfahrens a) wurden die in Tabelle 1 genannten Verbindungen der Formel (I-a) erhalten:

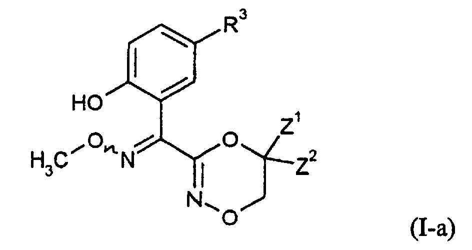

**Tabelle 1**

| Verbindung | R³ | Z¹ | Z² | F(°C) | logP | NMR* |
|---|---|---|---|---|---|---|
| 2 | -C₂H₅ | -H | -H | | 1,88 | 1,23 (m, 3H); 2,62 (m, 2H; 4,10 (s 3H); 4,21 (m, 2H); 4,49 (m, 2H) |
| 3 | -CH₃ | -H | -H | | 1,52 | 2,29 (s, 3H); 4,10 (s 3H); 4,21 (m, 2H); 4,49 (m, 2H) |
| 4 | -H | -CH₃ | -H | 136 | 1,49 | |
| 5 | -H | -C₂H₅ | -H | 134 | 1,82 | |
| 6 | -H | -CH₃ | -CH₃ | | 1,68 | |

| | | | | | | |
|---|---|---|---|---|---|---|
| *) Die ¹H-NMR-Spektren wurden in Deuterochloroform (CDCl₃) oder Hexadeuterodimethylsulfoxid (DMSO-d₆) mit Tetramethylsilan (TMS) als innerem Standard aufgenommen. Angegeben ist die chemische Verschiebung als δ-Wert in ppm. | | | | | | |

Analog Beispiel 1, Verbindung (II-1), sowie entsprechend der allgemeinen Beschreibung des erfindungsgemäßen Verfahrens b) wurden die in Tabelle 2 genannten Verbindungen der Formel (II-a) erhalten:

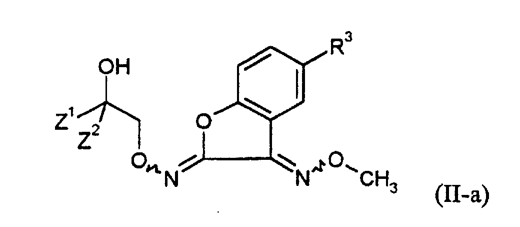

**Tabelle 2**

| Verbindung | R³ | Z ¹ | Z² | F (°C) | logP |
|---|---|---|---|---|---|
| (II-2) | -CH₃ | -H | -H | | 2,10 |
| (II-3) | -C₂H₅ | -H | -H | | 2,46 |
| (II-4) | -H | -H | -CH₃ | 81 | 2,04 |
| (II-5) | -H | -CH3 | -CH3 | 72 | 2,27 |
| (II-6) | -H | -H | -C2H5 | 74 | 2,39 |

## Patentansprüche

1. Verfahren zur Herstellung von Verbindungen der Formel 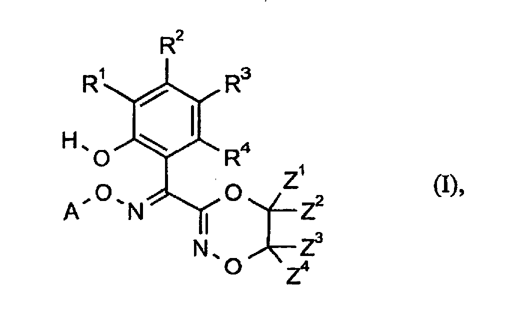 in welcher
A für Methyl, Ethyl, n- oder i-Propyl steht und
R¹, R², R³ und R⁴ gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen steht, und
Z¹, Z², Z³ und Z⁴ gleich oder verschieden sind und unabhängig voneinander für Wasserstoff, Alkyl oder Hydroxyalkyl mit jeweils 1 bis 4 Kohlenstoffatomen oder Halogenalkyl mit 1 bis 4 Kohlenstoffatomen und 1 bis fünf gleichen oder verschiedenen Halogenatomen stehen, oder
Z¹ und Z² oder Z¹ und Z³ oder Z³ und Z⁴ gemeinsam mit den jeweiligen Kohlenstoffatomen, an die sie gebunden sind, einen cycloaliphatischen Ring mit fünf, sechs oder sieben Kohlenstoffatomen bilden,
**dadurch gekennzeichnet, daß** man a) O-Hydroxyethyl-O'-alkyl-benzofurandiondioxime der Formel 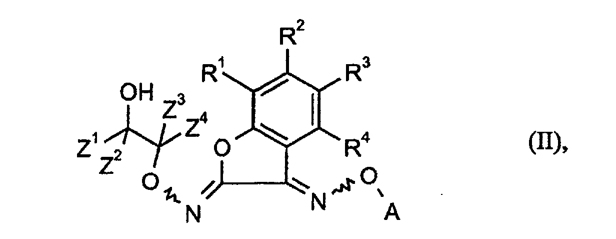 in welcher
A, R¹, R², R³, R⁴, Z¹, Z², Z³ und Z⁴ die oben angegebenen Bedeutungen haben,
gegebenenfalls in Gegenwart eines Verdünnungsmittels und gegebenenfalls in Gegenwart einer Base oder einer Säure, umlagert, und die erhaltenen Produkte, die gegebenenfalls als Gemische von Stereoisomeren vorliegen, gegebenenfalls zu den gewünschten E-Isomeren, gegebenenfalls in Gegenwart eines Verdünnungsmittels und gegebenenfalls in Gegenwart einer Säure, umisomerisiert.

2. Verfahren gemäß Anspruch 1, **dadurch gekennzeichnet, daß** man Verbindungen der Formel (I) herstellt, in welcher
A für Methyl oder Ethyl steht und
R¹, R², R³ und R⁴ gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n-oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl steht,
Z¹, Z², Z³ und Z⁴ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
Z¹ und Z² oder Z¹ und Z³ oder Z³ und Z⁴ gemeinsam mit den jeweiligen Kohlenstoffatomen, an die sie gebunden sind, einen cycloaliphatischen Ring mit fünf, sechs oder sieben Kohlenstoffatomen bilden.

3. Verbindungen der Formel (II) 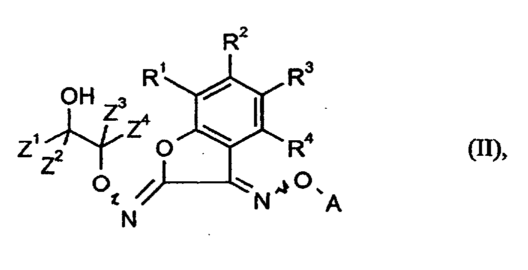 in welcher
A, R¹, R², R³, R⁴, Z¹, Z², Z³ und Z⁴ die in Anspruch 1 angegebenen Bedeutungen haben.

4. Verfahren zur Herstellung von Verbindungen der Formel (II) wie in Anspruch 3 definiert, **dadurch gekennzeichnet, daß** man
b) O-Alkyl-benzofurandiondioxime der Formel (III) 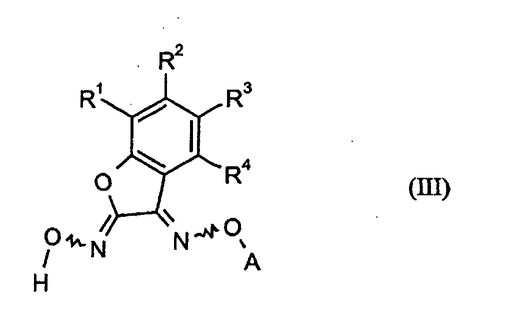 in welcher
A, R¹, R², R³ und R⁴ die in Anspruch 1 angegebenen Bedeutungen haben,
mit einem Ethanderivat der Formel (IV), 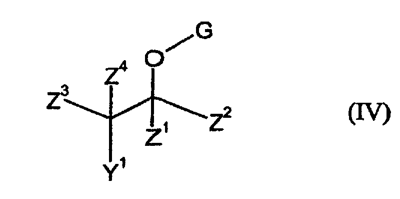 in welcher
Y¹ für Halogen, Alkylsulfonyloxy, Arylsulfonyloxy oder Alkanoyloxy steht und
G für Wasserstoff steht, oder
Y¹ und G durch eine Einfachbindung miteinander verbunden sind, wobei
Y¹ für Sauerstoff steht und
G für 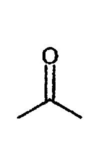 steht, oder
Y¹ und G gemeinsam für eine Einfachbindung stehen und
Z¹, Z², Z³ und Z⁴ die oben angegebenen Bedeutungen haben,
gegebenenfalls in Gegenwart eines Verdünnungsmittel, und gegebenenfalls in Gegenwart einer Base, umsetzt.

5. Verbindungen der allgemeinen Formel (III) 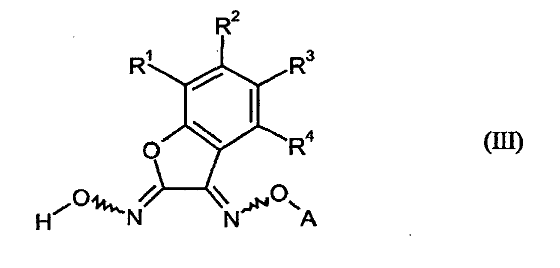 in welcher
A, R¹, R², R³, und R⁴ die in Anspruch 1 angegebenen Bedeutungen haben.

6. Verbindungen der allgemeinen Formel (V) 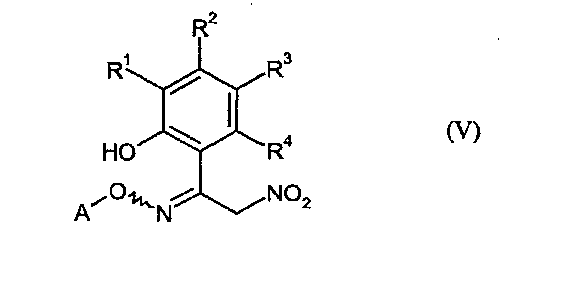 in welcher
A, R¹, R², R³ und R⁴ die in Anspruch 1 angegebenen Bedeutungen haben.

7. Verbindungen der allgemeinen Formel 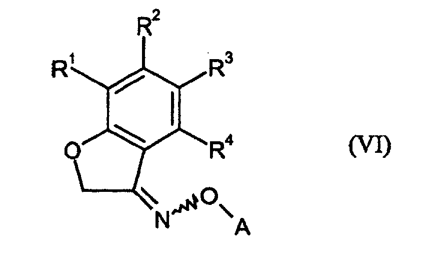 in welcher
A, R¹, R², R³ und R⁴ die in Anspruch 1 angegebenen Bedeutungen haben.

8. Verbindungen der allgemeinen Formel (IX) 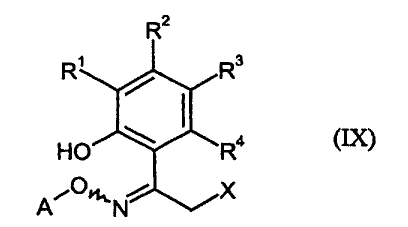 in welcher
A, R¹, R², R³ und R⁴ die in Anspruch 1 angegebenen Bedeutungen haben
und
X für Halogen steht.

## Claims

1. Process for preparing compounds of the formula 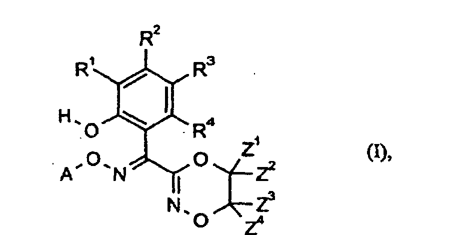 in which
A represents methyl, ethyl, n- or i-propyl and
R¹, R², R³ and R⁴ are identical or different and each represents independently of the others hydrogen, halogen, cyano, nitro, or represents alkyl, alkoxy, alkylthio, alkylsulphinyl or alkylsulphonyl having in each case 1 to 6 carbon atoms, each of which is optionally substituted by 1 to 5 halogen atoms, and
Z¹, Z², Z³ and Z⁴ are identical or different and each represents independently of the others hydrogen, alkyl or hydroxyalkyl having in each case 1 to 4 carbon atoms or halogenoalkyl having 1 to 4 carbon atoms and 1 to five identical or different halogen atoms, or
Z¹ and Z², or Z¹ and Z³, or Z³ and Z⁴, join with the respective carbon atoms to which they are attached to form a cycloaliphatic ring of five, six or seven carbon atoms,
**characterized in that** a) O-hydroxyethyl-O'-alkylbenzofurandione dioximes of the formula 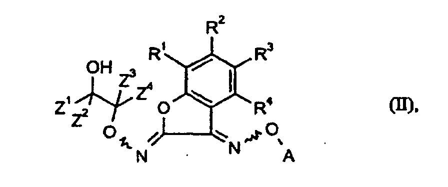 in which
A, R¹, R², R³, R⁴, Z¹, Z², Z³ and Z⁴ are each as defined above,
are rearranged, if appropriate in the presence of a diluent and, if appropriate, in the presence of a base or an acid, and the products obtained, which may be present as mixtures of stereoisomers, are, if appropriate, isomerized to give the desired E isomers, if appropriate in the presence of a diluent and, if appropriate, in the presence of an acid.

2. Process according to Claim 1, **characterized in that** compounds of the formula (I) are prepared in which
A represents methyl or ethyl and
R¹, R², R³ and R⁴ are identical or different and each represents independently of the others hydrogen, fluorine, chlorine, bromine, cyano, nitro, methyl, ethyl, n- or i-propyl, n-, i-, s- or t-butyl, methoxy, ethoxy, n- or i-propoxy, methylthio, ethylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl,
Z¹, Z², Z³ and Z⁴ are identical or different and each represents independently of the others hydrogen, methyl, ethyl, n- or i-propyl, n-, i-, s- or t-butyl, hydroxymethyl, trifluoromethyl or trifluoroethyl, or
Z¹ and Z², or Z¹ and Z³, or Z³ and Z⁴, join with the respective carbon atoms to which they are attached to form a cycloaliphatic ring of five, six or seven carbon atoms.

3. Compounds of the formula (II) 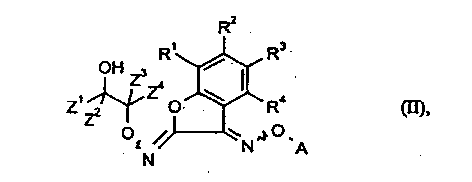 in which
A, R¹, R², R³, R⁴, Z¹, Z², Z³ and Z⁴ are each as defined in Claim 1.

4. Process for preparing compounds of the formula (II) as defined in Claim 3, **characterized in that**
b) O-alkyl-benzofurandione dioximes of the formula (III) 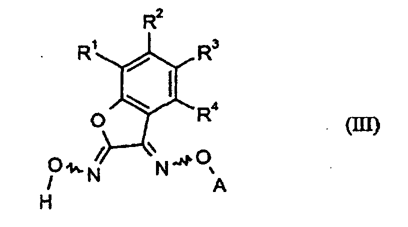 in which
A, R¹, R², R³ and R⁴ are each as defined in Claim 1,
are reacted with an ethane derivative of the formula (IV) 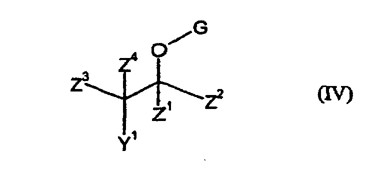 in which
Y¹ represents halogen, alkylsulphonyloxy, arylsulphonyloxy or alkanoyloxy and
G represents hydrogen, or
Y¹ and G are linked by a single bond, where
Y¹ represents oxygen and
G represents 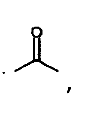 or
Y¹ and G together represent a single bond and
Z¹, Z², Z³ and Z⁴ are each as defined above,
if appropriate in the presence of a diluent and, if appropriate, in the presence of a base.

5. Compounds of the formula (III) 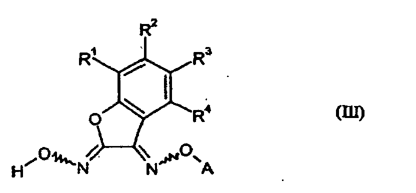 in which
A, R¹, R², R³ and R⁴ are each as defined in Claim 1.

6. Compounds of the formula (V) 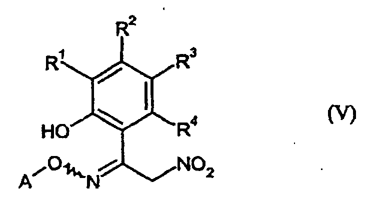 in which
A, R¹, R², R³ and R⁴ are each as defined in Claim 1.

7. Compounds of the formula 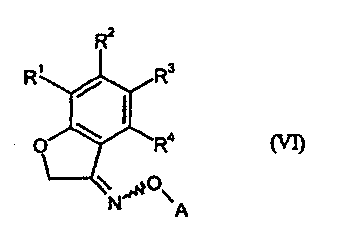 in which
A, R¹, R², R³ and R⁴ are each as defined in Claim 1.

8. Compounds of the formula (IX) 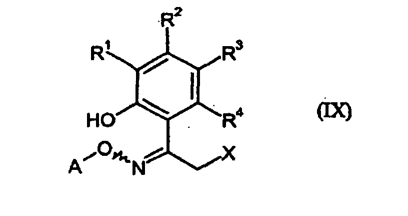 in which
A, R¹, R², R³ and R⁴ are each as defined in Claim 1
and
X represents halogen.

## Revendications

1. Procédé de production de composés de formule 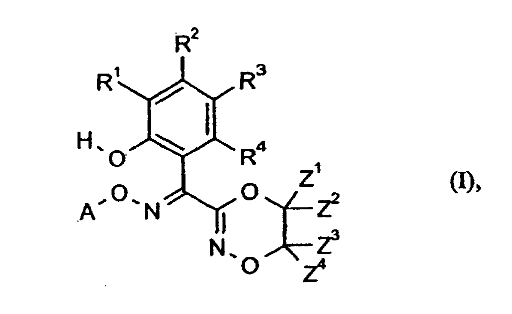 dans laquelle
A est un reste méthyle, éthyle, n-propyle ou isopropyle et
R¹, R², R³ et R⁴ sont identiques ou différents et représentent, indépendamment les uns des autres l'hydrogène, un halogène, un groupe cyano, nitro, un reste alkyle, alkoxy, alkylthio, alkylsulfinyle ou alkylsulfonyle ayant chacun 1 à 6 atomes de carbone et dont chacun est éventuellement substitué par 1 à 5 atomes d'un halogène, et
Z¹, Z², Z³ et Z⁴ sont identiques ou différents et représentent indépendamment les uns des autres l'hydrogène, un reste alkyle ou un reste hydroxyalkyle ayant chacun 1 à 4 atomes de carbone ou un reste halogénalkyle ayant 1 à 4 atomes de carbone et 1 à 5 atomes d'un même halogène ou d'halogènes différents, ou bien
Z¹ et Z² ou Z¹ et Z³ ou Z³ et Z⁴ forment conjointement avec les atomes de carbone auxquels ils sont liés un noyau cycloaliphatique ayant 5, 6 ou 7 atomes de carbone,
**caractérisé en ce que** a) on transpose des O-hydroxyéthyl-O'-alkyl-benzofuranne-dione-dioximes de formule 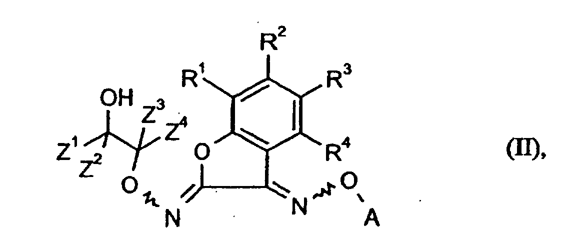 dans laquelle
A, R¹, R², R³, R⁴, Z¹, Z², Z³ et Z⁴ ont les définitions indiquées ci-dessus,
le cas échéant en présence d'un diluant et en présence éventuelle d'une base ou d'un acide, et on transforme éventuellement les produits obtenus, qui sont le cas échéant sous forme de mélanges de stéréoisomères, en les E-isomères souhaités, éventuellement en présence d'un diluant et éventuellement en présence d'un acide.

2. Procédé suivant la revendication 1, **caractérisé en ce qu'**on prépare des composés de formule (I) dans laquelle
A est un reste méthyle ou éthyle et
R¹, R², R³ et R⁴ sont identiques ou différents et représentent chacun, indépendamment les uns des autres, l'hydrogène, le fluor, le chlore, le brome, un groupe cyano, un groupe nitro, un reste méthyle, éthyle, n-propyle, isopropyle, n-butyle, isobutyle, sec.-butyle, tertiobutyle, méthoxy, éthoxy, n-propoxy, isopropoxy, méthylthio, éthylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhyltio, trifluorométhylthio, trifluorométhylsulfinyle ou trifluorométhylsulfonyle,
Z¹, Z², Z³ et Z⁴ sont identiques ou différents et représentent, indépendamment les uns des autres, l'hydrogène, un reste méthyle, éthyle, n-propyle, isopropyle, n-butyle, isobutyle, sec.-butyle, tertiobutyle, hydroxyméthyle, trifluorométhyle ou trifluoréthyle, ou bien
Z¹ et Z² ou Z¹ et Z³ ou Z³ et Z⁴ forment conjointement avec les atomes de carbone auxquels ils sont liés un noyau cycloaliphatique ayant 5, 6 ou 7 atomes de carbone.

3. Composés de formule (II) 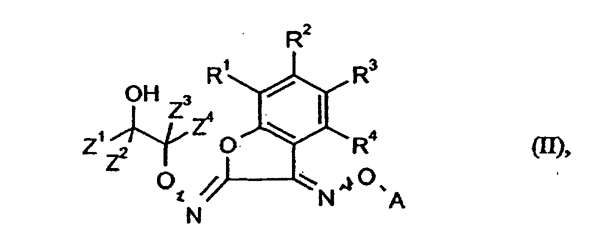 dans laquelle
A, R¹, R², R³, R⁴, Z¹, Z², Z³ et Z⁴ ont les définitions indiquées dans la revendication 1.

4. Procédé de production de composés de formule (II) telle que définie dans la revendication 3, **caractérisé en ce que**
b) on fait réagir des O-alkyl-benzofuranne-dione-dioximes de formule (III) 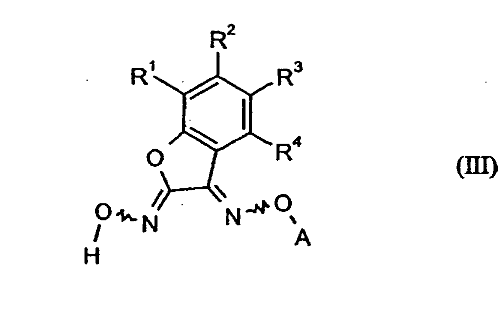 dans laquelle
A, R¹, R², R³ et R⁴ ont les définitions indiquées dans la revendication 1,
avec un dérivé d'éthane de formule (IV) 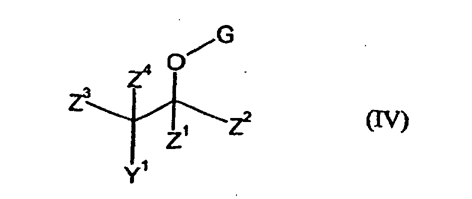 dans laquelle
Y¹ représente un halogène, un reste alkylsulfonyloxy, arylsulfonyloxy ou alcanoyloxy et
G représente l'hydrogène, ou bien
Y¹ et G sont liés ensemble par une liaison simple, auquel cas
Y¹ est l'oxygène et
G représente 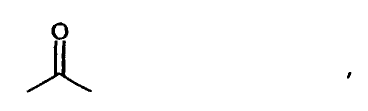 ou bien
Y¹ et G forment ensemble une liaison simple et
Z¹, Z², Z³ et Z⁴ ont les définitions indiquées ci-dessus,
éventuellement en présence d'un diluant et, le cas échéant, en présence d'une base.

5. Composés de formule générale (III) 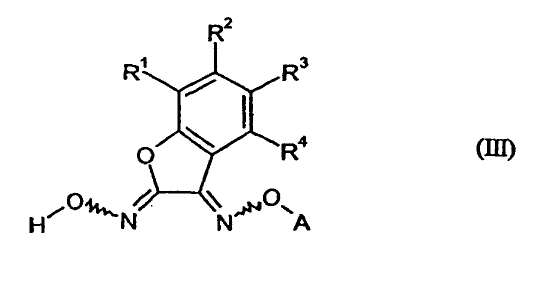 dans laquelle
A, R¹, R², R³ et R⁴ ont les définitions indiquées dans la revendication 1.

6. Composés de formule générale (V) 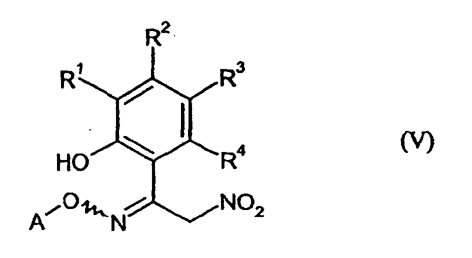 dans laquelle
A, R¹, R², R³ et R⁴ ont les définitions indiquées dans la revendication 1.

7. Composés de formule générale 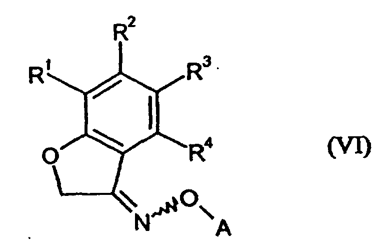 dans laquelle
A, R¹, R², R³ et R⁴ ont les définitions indiquées dans la revendication 1.

8. Composés de formule générale (IX) 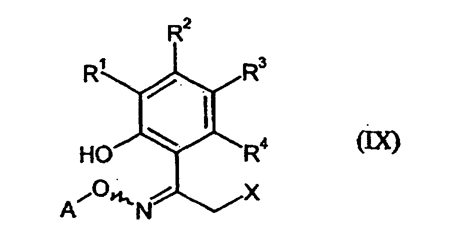 dans laquelle
A, R¹, R², R³ et R⁴ ont les définitions indiquées dans la revendication 1
et
X est un halogène.
